(19) Europäisches Patentamt
European Patent Office
Office européen des brevets

(11) **EP 2 704 048 A2**

(12) **EUROPEAN PATENT APPLICATION**

(43) Date of publication:
**05.03.2014 Bulletin 2014/10**

(51) Int Cl.:
***G06F 19/00*** *(2011.01)*

(21) Application number: **13182141.5**

(22) Date of filing: **29.08.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.08.2012 JP 2012192100**

(71) Applicant: **Sumitomo Rubber Industries, Ltd.**
**Kobe-shi, Hyogo 651-0072 (JP)**

(72) Inventor: **Ueno, Shinichi**
**Kobe-shi, Hyogo 651-0072 (JP)**

(74) Representative: **Manitz, Finsterwald & Partner GbR**
**Martin-Greif-Strasse 1**
**80336 München (DE)**

(54) **Method for simulating polymer material**

(57)    A computer implemented method for simulating a polymer material comprises a simulation process in which a molecular dynamics calculation is performed about polymer models and filler models. The simulation process comprises: a first calculation process in which the molecular dynamics calculation is performed under such condition that, between particles of each polymer model, a first coupling potential not limiting the interparticle distance is defined; and a subsequent second calculation process in which the molecular dynamics calculation is performed under such condition that, between the particles of each polymer model, a second coupling potential P2 limiting the maximum allowable interparticle distance to a predetermined value is defined.

FIG.2

## Description

### Background of the Invention

The present invention relates to a computer implemented method for simulating a polymer material capable of continuing a relaxation calculation without calculation failure caused by entanglement of polymer chain models.

In general, a rubber compound used in a pneumatic tire contains reinforcing filler such as carbon black and silica. For example, if a silica-rich compound is used as a tread rubber of a pneumatic tire, an internal energy loss of the tread rubber is decreased and the tire performance, e.g. rolling resistance may be improved. Such silica-rich compound contains a modifying agent to increase the affinity of the base rubber or elastomer to the silica filler. If the affinity is low, the strength of the rubber compound is decreased with the increase in the content of the filler.

In recent years, the use of a computer simulation is proposed in order to expedite the development process of such a rubber compound.

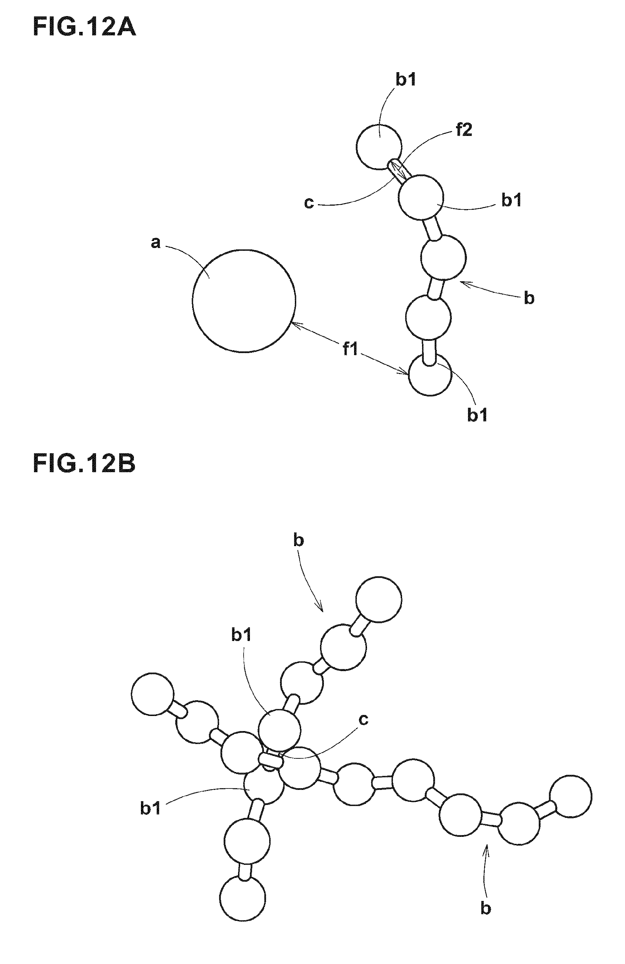

According to a widely employed coarse-grained molecular dynamic method, as shown in Fig. 12A,

a polymer model (b) of a polymer chain of the polymer material is defined by spherical particles (b1),

coupling potentials f2 are defined between the particles (b1) of the polymer model (b) as the bonds (c) therebetween, a filler model (a) of the filler is defined by a spherical particle, and

potentials f1 are defined between the particle of the filler model (a) and the particles (b1) the polymer model (b), and then a molecular dynamics calculation is performed.

In order to simulate the molecular motion of the polymer material, the coupling potentials f2 between the particles (b1) are defined such that the interparticle distance is not allowed to increase over a predetermined allowable maximum distance.

On the other hand, during the molecular dynamics calculation (relaxation calculation), there is a possibility that the moved polymer models (b) are entangled as shown in Fig. 12B.

If the polymer models (b) are entangled, since the distance between the particles (b1) at the entangled position is not allowed to increase over the allowable maximum distance, there is a possibility that the polymer models (b) can not be disentangled, and as a result, the force acting between the polymer models (b) is extremely increased by the molecular dynamics calculation, and a calculation failure to stop the molecular dynamics calculation is caused. Such calculation failure is liable to occur when the relaxation calculation starts from a concentrated or aggregated state of the polymer models.

If the calculation failure is caused, it becomes impossible to make the relaxation of the polymer models which is important for accurate simulation and analysis.

### Summary of the Invention

It is therefore an object of the present invention to provide a computer implemented method for simulating a polymer material in which a molecular dynamics calculation of polymer models can be continued, without calculation failure due to entanglements of the polymer models, to achieve an accurate relaxation.

According to the present invention, a computer implemented method for simulating a polymer material, comprises

a process in which polymer models of the polymer material are defined,

a process in which filler models of the filler are defined, and

a simulation process in which a molecular dynamics calculation is performed on the polymer models and the filler models arranged in a predetermined virtual space, wherein

the polymer model comprises a plurality of polymer particles and bonds between the polymer particles,

the bond is defined by a coupling potential P,

the coupling potential P includes a first coupling potential P1 and a second coupling potential P2, the second coupling potential P2 not allowing any interparticle distances more than a predetermined allowable maximum distance, and the first coupling potential P1 having no allowable maximum distance for the interparticle distance, and

the simulation process comprises a first calculation process and a subsequent second calculation process,

in the first calculation process, the molecular dynamics calculation is performed under such condition that the first coupling potentials P1 are defined between the polymer particles, and

in the second calculation process, the molecular dynamics calculation is performed under such condition that the second coupling potentials P2 are defined between the polymer particles.

Therefore, in the first calculation process, since the interparticle distance between the polymer particles is not limited, even if the polymer models are entangled, the polymer models are easily disentangled. Therefore, the acting force between the entangled polymer models can be prevented from becoming extremely increased. Thus, the occurrence of calculation failure can be effectively prevented.

In this way, through the first calculation process, the polymer particles are well relaxed even if the calculation is started from a concentrated or aggregated state.

Starting from such relaxed state, the molecular dynamics calculation of the second calculation process is performed without allowing the interparticle distance to increase over the allowable maximum distance. Therefore, in the second calculation process, the relaxation similar to the actual molecular motion of the polymer material can be made to provide accurate simulation results.

The simulation method according to the present invention may have the following features (a) and/or (b):

(a) the virtual space has a pair of parallelly-opposed wall surfaces, and the filler models are respectively defined by the parallelly-opposed wall surfaces;
(b) the first coupling potential P1 is defined by the following equation (1),

the second coupling potential P2 is defined by the sum of a repulsive potential R defined by the following equation (2) and an attractive potential G defined by the following equation (3):

$$P1 = 0.5k(r_{ij} - r')^2 \qquad --(1)$$

$$P2 = R + G$$

$$R = \begin{cases} 4\varepsilon\left[\left(\dfrac{\sigma}{r_{ij}}\right)^{12} - \left(\dfrac{\sigma}{r_{ij}}\right)^6 + \dfrac{1}{4}\right] & if\ r_{ij} < 2^{\frac{1}{6}}\sigma \\ 0 & if\ r_{ij} \geq 2^{\frac{1}{6}}\sigma \end{cases} \qquad ---(2)$$

$$G = \begin{cases} -0.5kr_0^{\ 2}\ln\left[1 - \left(\dfrac{r_{ij}}{r_0}\right)^2\right] & if\ r_{ij} < r_0 \\ \infty & if\ r_{ij} \geq r_0 \end{cases} \qquad ---(3)$$

wherein

k: the spring constant between the particles concerned,
$r_{ij}$: the interparticle distance between the centers of the particles concerned,
r': the equilibrium distance between the centers of the particles concerned,
$r_0$: a predetermined allowable maximum distance between the centers of the particles concerned.
$\varepsilon$: a coefficient for the intensity of the repulsive potential R between the particles concerned,
$\sigma$: a coefficient for adjusting the threshold of the distance $r_{ij}$.

Brief Description of the Drawings

Fig. 1 is a perspective view of a computer system for implementing a simulation method as an embodiment of the present invention.

Fig. 2 is a flow chart of the simulation method.

Fig. 3 is a diagram for explaining repulsive potentials R defined between polymer models.

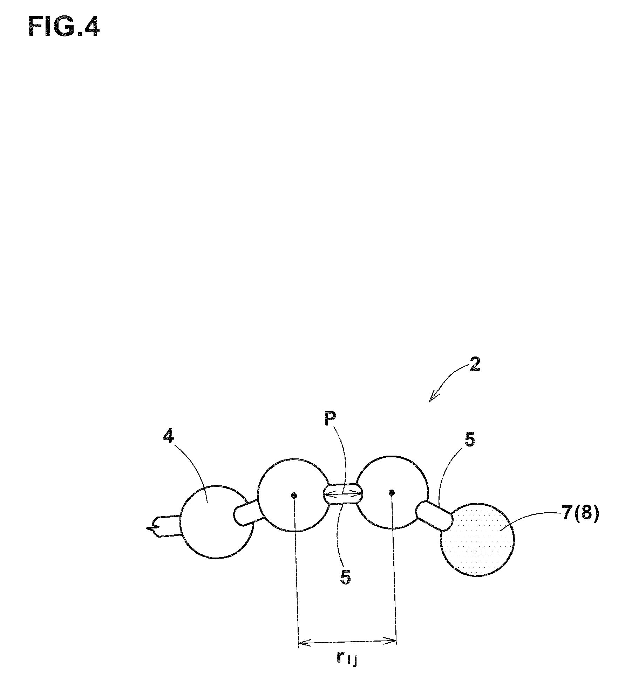

Fig. 4 shows a part of the polymer model.

Fig. 5 is a perspective view of an example of the virtual space.

Fig. 6 is a diagram for explaining interactive potentials between the filler model and the polymer model.

Fig. 7 shows an example of the initial state of the polymer models disposed in the virtual space.

Fig. 8 is a flow chart of an example of the simulation process.

Fig. 9A shows polymer models entangled during the first calculation process.

Fig. 9B shows the polymer models disentangled during the first calculation process.

Fig. 10 shows a relaxed state of the polymer models in the virtual space after the first calculation process.

Fig. 11 shows only two of the polymer models in the virtual space after the second calculation process.

Fig. 12A shows a conventional filler model and polymer model.

Fig. 12B shows entangled polymer models for explaining the problem to be solved.

## Description of the Preferred Embodiments

Embodiments of the present invention will now be described in detail in conjunction with accompanying drawings.

The simulation method according to the present invention is to simulate a polymer material or mixture including a polymer and a filler and optionally a modifying agent to increase the affinity of the polymer to the filler and can be used to evaluate or estimate the reaction between the filler and polymer and thus, can be used to estimate the characteristics of the cured polymer material.

Here, the filler may be any kind of filler including carbon black, silica, alumina and the like. The polymer may be any kind of polymer including rubber, elastomer, resin and the like. The modifying agent may be any kind of modifying agent having a functional group which is an atom group including a hydroxyl group or carbonyl group.

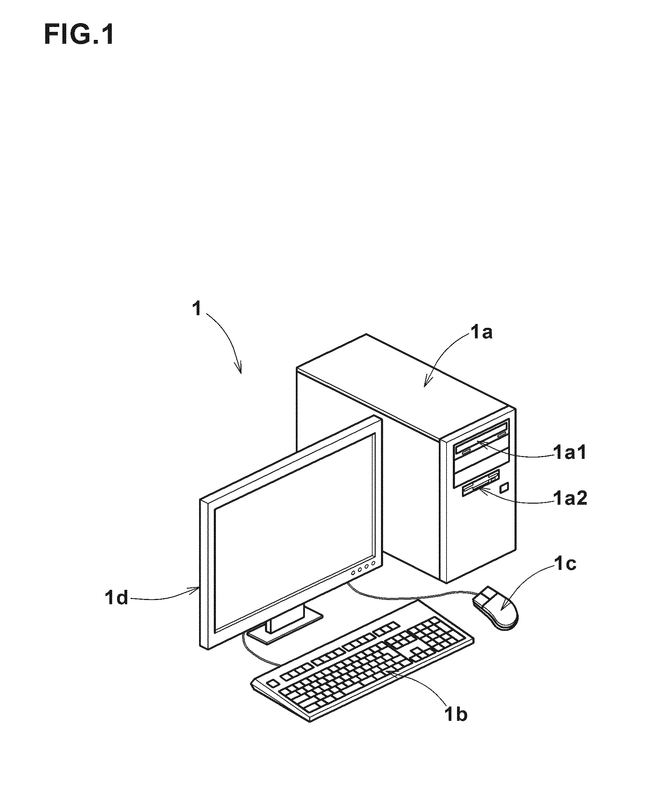

As shown in Fig. 1 for example, the computer system 1 implementing the simulation method comprises a main body 1a, a keyboard 1b, a mouse 1c and a display 1d. The main body 1a comprises an arithmetic processing unit (CPU), memory, storage devices such as magnetic disk, disk drives 1a1 and 1a2 and the like. In the storage device, programs/ software for carrying out the simulating method are stored.

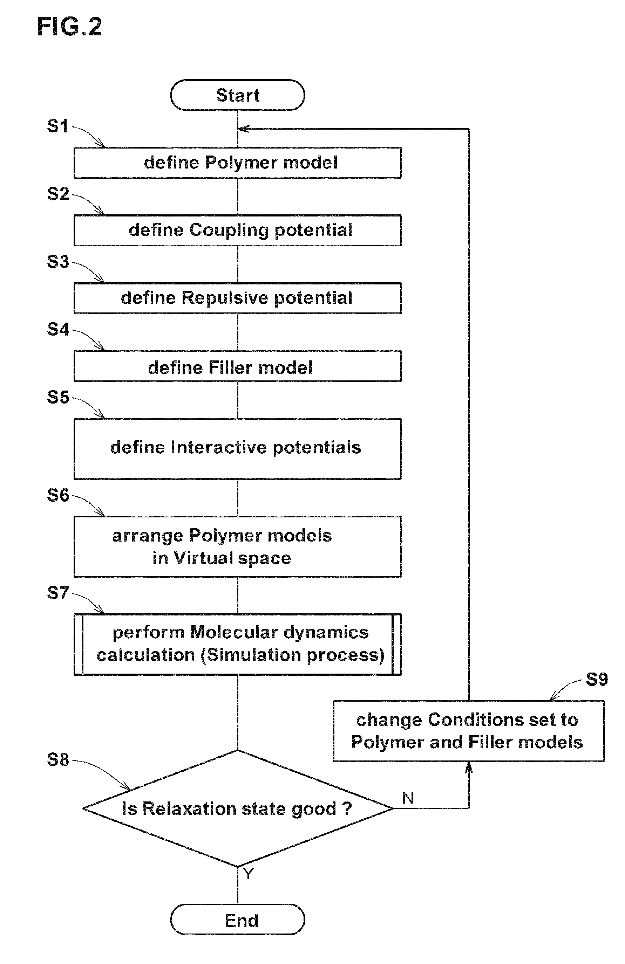

Fig. 2 shows a flowchart of the simulation method as an embodiment of the present invention.

This flowchart is just for illustrative purposes. It is not always necessary to perform these processes in this order.

In a process S1, a plurality of polymer models 2 of the polymer are defined.

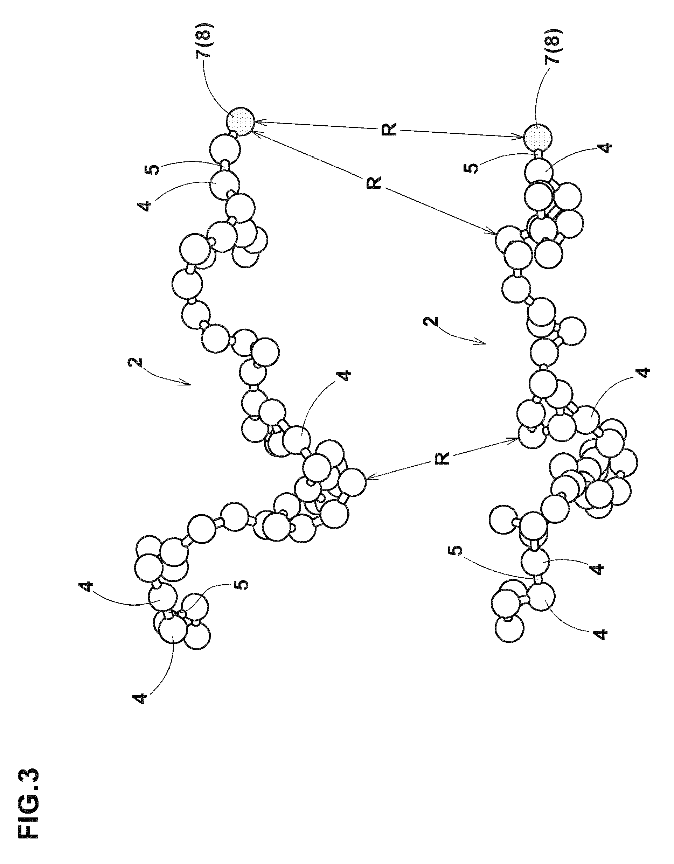

As shown in Fig. 3, each of the polymer models 2 comprises a plurality of polymer particles 4 (for example, 10 to 5000 polymer particles 4) and bonds 5 between the polymer particles 4.

The polymer model 2 represents a three-dimensional straight-chain structure of the polymer.

In this embodiment, the polymer material includes the above-mentioned modifying agent, therefore, each of the polymer models 2 includes at least one modifying agent model 7 of the modifying agent.

As shown in Fig. 3, each of the modifying agent models 7 is represented by at least one particle 8. The particle 8 of the modifying agent model 7 is bonded with the polymer particle 4 through the bond 5.

The polymer model 2 is, of course, a set of numerical data (inclusive of data on the mass, volume, diameter and initial stage coordinates of each particle 4, 8) to be used in a molecular dynamics calculation, and the numerical data are stored in the computer 1.

In a process S2, each of the bonds 5 is defined by a coupling potential P.

* First and second coupling potentials P1 and P2

In this invention, two kinds of coupling potentials P (first coupling potential P1 and second coupling potential P2) are employed as explained later. For the sake of convenience of the subsequent explanation, the two kinds of potentials P1 and P2 are described in advance.

The first coupling potential P1 is defined by the following equation (1) as Harmonic potential.

$$P1 = 0.5k(r_{ij}-r')^2 \qquad \text{--(1)}$$

wherein

k: the spring constant between the particles concerned,
$r_{ij}$: the interparticle distance between the centers of the particles concerned and
r': the equilibrium distance between the centers of the particles concerned.

Incidentally, the Harmonic potential is such a potential that exerts a restoring force almost proportional to the amount of displacement from the equilibrium distance r' like a restoring force of a linear spring.

By the equation (1), when the interparticle distance $r_{ij}$ becomes equal to the equilibrium distance r', the first coupling potential P1 becomes zero, and no force acts between the particles concerned.

When the interparticle distance $r_{ij}$ is more than the equilibrium distance r', the first coupling potential P1 becomes increased with the increase in the interparticle distance $r_{ij}$. When the interparticle distance $r_{ij}$ is less than the equilibrium distance r', the first coupling potential P1 becomes increased with the decrease in the interparticle distance $r_{ij}$. Thus, by the first coupling potential P1, a force restoring the interparticle distance $r_{ij}$ to the equilibrium distance r' takes place between the particles concerned.

The second coupling potential P2 is defined by the sum of a repulsive potential R and an attractive potential G. The repulsive potential R is defined by the following equation (2). The attractive potential G is defined by the following equation (3).

$$P2 \ = \ R \ + \ G$$

$$R = \begin{cases} 4\varepsilon \left[ \left( \dfrac{\sigma}{r_{ij}} \right)^{12} - \left( \dfrac{\sigma}{r_{ij}} \right)^{6} + \dfrac{1}{4} \right] & if \ r_{ij} < 2^{\frac{1}{6}} \sigma \\ 0 & if \ r_{ij} \geq 2^{\frac{1}{6}} \sigma \end{cases} \qquad ---(2)$$

$$G = \begin{cases} -0.5 k r_0^{2} \ln \left[ 1 - \left( \dfrac{r_{ij}}{r_0} \right)^{2} \right] & if \ r_{ij} < r_0 \\ \infty & if \ r_{ij} \geq r_0 \end{cases} \qquad ---(3)$$

wherein

$r_{ij}$: the interparticle distance between the centers of the particles concerned,
$r_0$: a predetermined allowable maximum distance between the centers of the particles concerned,
k: the spring constant between the particles concerned,
$\varepsilon$: a coefficient for the intensity of the repulsive potential R between the particles concerned,
$\sigma$: a coefficient for adjusting the threshold of the distance $r_{ij}$. These coefficients and variable correspond to the parameters of the Lennard-Jones potential.

The repulsive potential R is increased with the decrease in the interparticle distance $r_{ij}$. The attractive potential G is increased with the increase in the interparticle distance $r_{ij}$. Accordingly, the second coupling potential P2 exerts a force which restores the interparticle distance $r_{ij}$ to a distance at which the repulsive potential R is balanced against the attractive potential G.

In the equation (3) used to define the second coupling potential P2, any suitable values may be set to the spring constant k and allowable maximum distance $r_0$ of the attractive potential G and the coefficient $\varepsilon$ for the intensity of the repulsive potential R.

In this embodiment, according to Non-patent document ("Dynamics of entangled linear polymer melts; A molecular-dynamics simulation" Journal of Chemical Physics, 92, 5057(1990)),

30 is set to the "k",

1.5 is set to the "$r_0$",

1.0 is set to the "$\varepsilon$" and

1.0 is set to the "$\sigma$".

On the other hand, in the above-mentioned equation (1) defined as the first coupling potential P1, any suitable values may be set to the spring constant k and the equilibrium distance r'. In this embodiment, in order that the first coupling potential P1 becomes comparable to the second coupling potential P2,

900 is set to the "k", and

0.9 is set to the "r'".

In the equation (3), when the interparticle distance $r_{ij}$ is the same as or more than the allowable maximum distance $r_0$, the attractive potential G is defined as infinity. By the second coupling potential P2, therefore, the interparticle distance $r_{ij}$ is not allowed to have any values more than the allowable maximum distance $r_0$.

In contrast, in the first coupling potential P1, any allowable maximum distance is not defined for the interparticle distance $r_{ij}$. Thus, when compared with the first coupling potential P1, it can be said that the second coupling potential P2 more accurately approximates to the molecular motion of the polymer material.

In order to place the polymer models 2, a virtual space 6 having a predetermined volume is defined. The virtual space 6 has a pair of parallelly-opposed wall surfaces 11 and 11 between which the polymer models 2 are disposed.

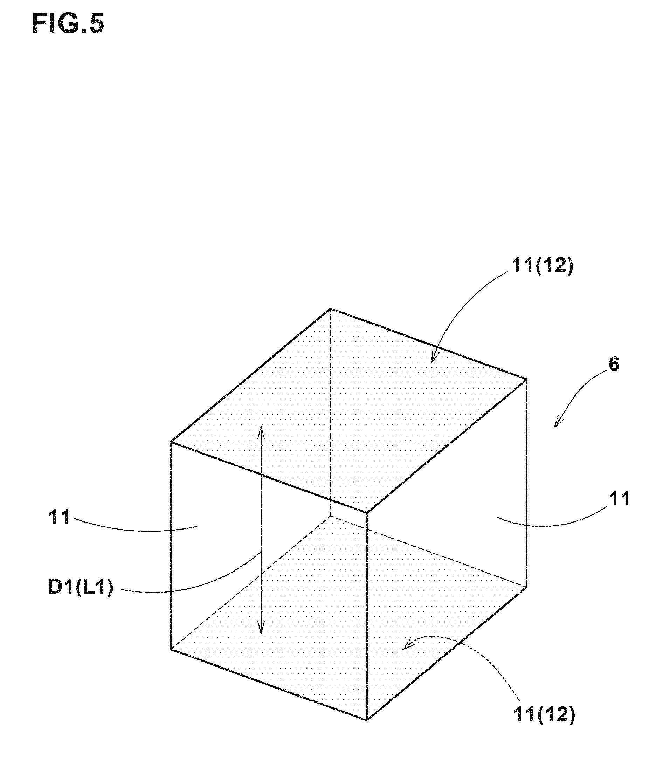

On the virtual space 6, there is defined a condition such that the polymer model 2 can not pass through the wall surface 11. In this example, as shown in Fig. 5, the shape of the virtual space 6 is a regular hexahedron having three pairs of parallelly-opposed wall surfaces 11.

It is desirable that the distance D1 (length L1 of a side) between the paired parallelly-opposed wall surfaces 11 measured perpendicularly thereto is not less than 2 times, preferably not less than 4 times the radius of inertia of the polymer model 2. Thereby, in the after-mentioned molecular dynamics calculation, it becomes possible to stably calculate the rotational motion of the polymer model 2 in the virtual space 6.

The volume of the virtual space 6 is set, in relation to the number of the particles placed in the virtual space 6, such that the particle number density becomes 0.85 per $\sigma^3$, for example according to the above-mentioned non-patent document.

Getting back to the flowchart shown in Fig. 2, in a process S4, a pair of filler models 12 and 12 are defined by a pair of the above-mentioned parallelly-opposed wall surfaces 11 and 11, respectively.

Namely, each filler model 12 is defined by a flat face instead of a particle (or spherical surface).

In the example shown in Fig. 5, the wall surfaces 11 and 11 which are parallelly-opposed in the up-and-down direction are used as filler models 12 and 12, respectively.

The paired filler models 12 are accordingly immovable with respect to the virtual space 6.

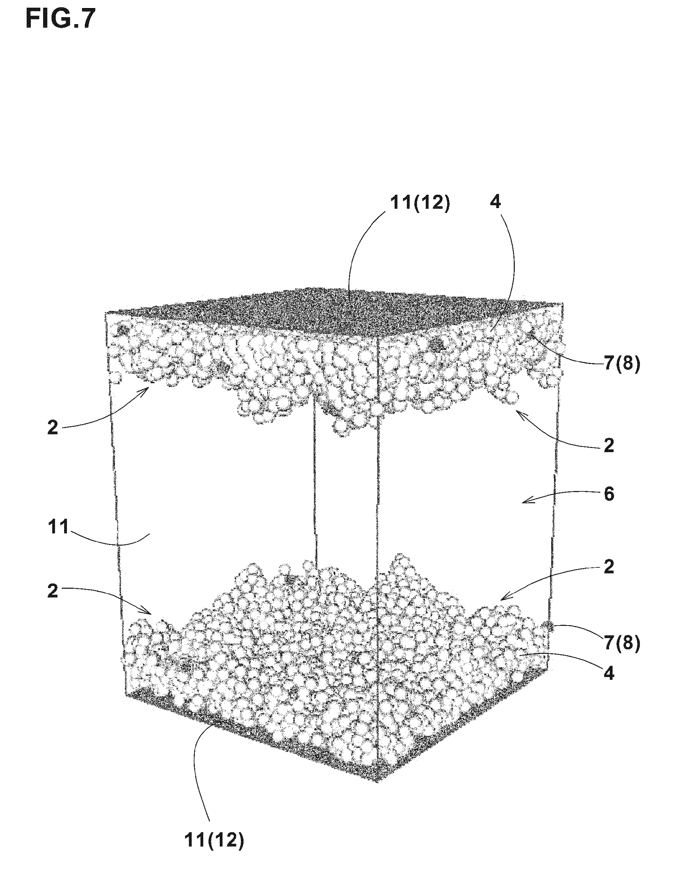

In a process S6, the polymer models 2 are arranged in the virtual space 6. For example, about 30 to 400 polymer models 2 are arranged in the virtual space 6. In this example, as shown in Fig. 7, about 200 to 400 polymer models 2 are arranged in a concentrated manner closely to both of the parallelly-opposed wall surfaces 11 (filler models 12).

In a process S3, between the polymer models 2, repulsive potentials R are defined. More specifically, as shown in Fig. 3, between each of the particles 4 of the polymer model 2 and each of the particles 4 of another polymer model 2, between the singe particle 8 or each of the particles 8 of the polymer model 2 and the single particle 8 or each of the particles 8 of another polymer model 2 and

between each of the particles 4 of the polymer model 2 and the single particle 8 or each of the particles 8 of another polymer model 2,

the repulsive potentials R are respectively defined.

In this embodiment, the repulsive potential R is defined by the above-mentioned equation (2).

As to the coefficients $\varepsilon$ and $\sigma$ of the repulsive potential R, the same values as explained above are preferably set thereto.

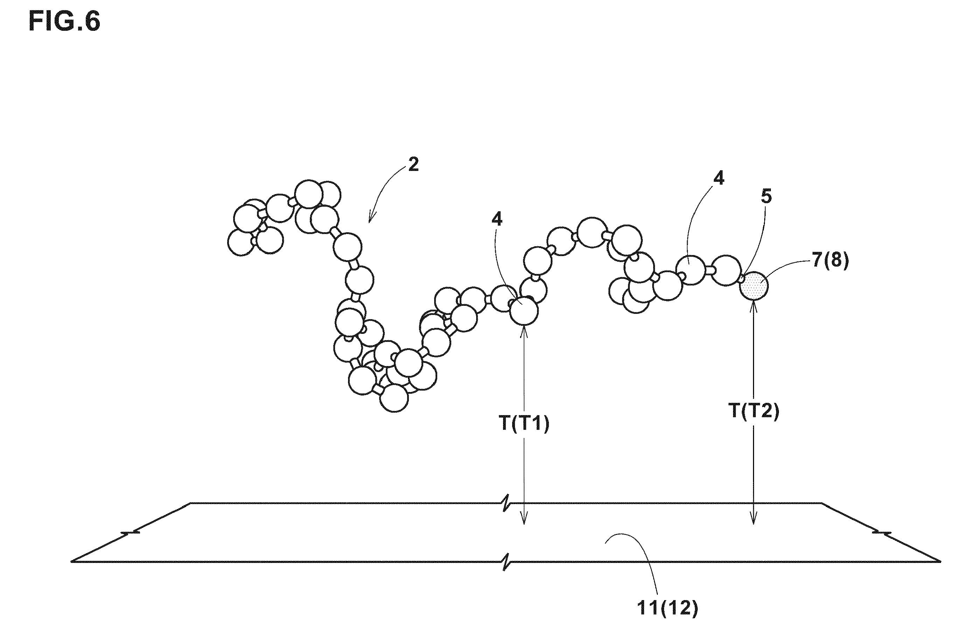

In a process S5, between the filler models 12 and the polymer models 2, interactive potentials T exerting an attractive force or a repulsive force are defined. More specifically, as shown in Fig. 6,

between each of the filler models 12 and each of the particles 4 and

between each of the filler models 12 and each of the particles 8, the interactive potentials T are respectively defined.

The interactive potential T is defined by the following equation (4).

$$T = \begin{cases} 4\pi\rho_{wall}\varepsilon_{wall}\left[\dfrac{1}{5}\left(\dfrac{\sigma_{wall}}{r}\right)^{10} - \dfrac{1}{2}\left(\dfrac{\sigma_{wall}}{r}\right)^{4}\right] & if\ r < r_c \\[2mm] 0 & if\ r \geq r_c \end{cases} \quad ---(4)$$

wherein

r: the distance between the filler model and the center of the particle 4 or 8 concerned,
$r_C$: a threshold of the distance,
$\rho_{wall}$: a coefficient relating to the areal density of the interactive potential T,
$\varepsilon_{wall}$: a coefficient relating to the intensity of the interactive potential T, and
$\sigma_{wall}$: a coefficient relating to the distance from the filler model (wall surface 11).

The interactive potential T defined by the equation (4) can be obtained by integrating the repulsive potential R defined by the equation (2) over the wall surface 11 (the filler model 12).
By the equation (4), when the distance r is more than the predetermined threshold $r_C$, the interactive potential T becomes zero.
When the distance r is less than $\sigma_{wall}$ X $2^{1/6}$, the interactive potential T exerts a repulsive force between the filler model 12 and the particle 4, 8 concerned.
When the distance r is more than $\sigma_{wall}$ X $2^{1/6}$, the interactive potential T exerts an attractive force between the filler model 12 and the particle 4, 8 concerned.
Thus, by the equation (4), an attractive force or repulsive force takes place between the filler model 12 and the particle 4, 8 concerned according to the distance r therebetween.
In the example shown in Fig. 6, the following interactive potentials T1 and T2 are defined.

T1: between filler model 12 and particle 4 (nonmodified)
T2: between filler model 12 and particle 8 (modified)

Any suitable values may be set to $\rho_{wall}$, $\sigma_{wall}$, $\varepsilon_{wall}$ and $r_C$ of the interactive potentials T1 and T2. In this embodiment, these parameters are set as follows. interactive potential T1:

$\rho_{wall}$ = 1.0
$\sigma_{wall}$ = 1.0
$\varepsilon_{wall}$ = 1.0
$r_C$ = 1.12
interactive potential T2:
$\rho_{wall}$ = 1.0
$\sigma_{wall}$ = 1.0
$\varepsilon_{wall}$ = 5.0
$r_C$ = 2.5

By setting the value of $\varepsilon_{wall}$ (=5) of the interactive potential T2 larger than the value of $\varepsilon_{wall}$ (=1) of the interactive potential T1 as above, the intensity of the attractive force or repulsive force between the filler model 12 and the particle 8 can increase more than that between the filler model 12 and the particle 4.
By setting 2.5 to the threshold $r_C$ for the interactive potential T2, in a relatively wide range of the distance r from $2^{1/6}$(=1.12) to 2.5, the interactive potential T2 can exert an attractive force between the filler model 12 and the particle 8.
By setting 1.12 to the threshold $r_C$ for the interactive potential T1, the interactive potential T1 exerts only a repulsive force between the filler model 12 and the particle 4. Accordingly, the particle 8 (modified) receives an attractive force dominantly than the particle 4 (nonmodified).
Thus, by setting the $\varepsilon_{wall}$ and $r_C$ of the interactive potentials T1 and TR2 as explained above, the affinity of the particle 8 to the filler model 12 can be defined as being more than the affinity of the particle 4 to the filler model 12.
In a simulation process S7, a relaxation of the particles 4 and 8 of the polymer models 2 between the filler models 12 is simulated by performing the molecular dynamics calculation.

In this example, on the assumption that the particles 4 and 8 of the polymer models 2 accords with classical dynamics, the Newton's equation of motion is applied to the molecular dynamics calculation. And the motion of the particles 4 and 8 are tracked at constant time interval. During the calculation, the number of the particles in the virtual space 6, and the temperature and the volume of the virtual space 6 are kept constant.

Since the filler model 12 is locked to a pair of the parallelly-opposed wall surfaces 11 of the virtual space 6, the relaxation calculation can be performed, targeting at the polymer model 2 only. Therefore, the computational time is remarkable reduced when compared with a relaxation calculation performed targeting at both of the polymer models and movable filler models.

Further, the direction of the interactive potential field caused by the filler model 12 is one direction perpendicular to the wall surface 11, in contrast to a spherical filler model resulting in radial directions or all directions. Therefore, the relaxation calculation becomes relatively simple and the computational time may be further reduced.

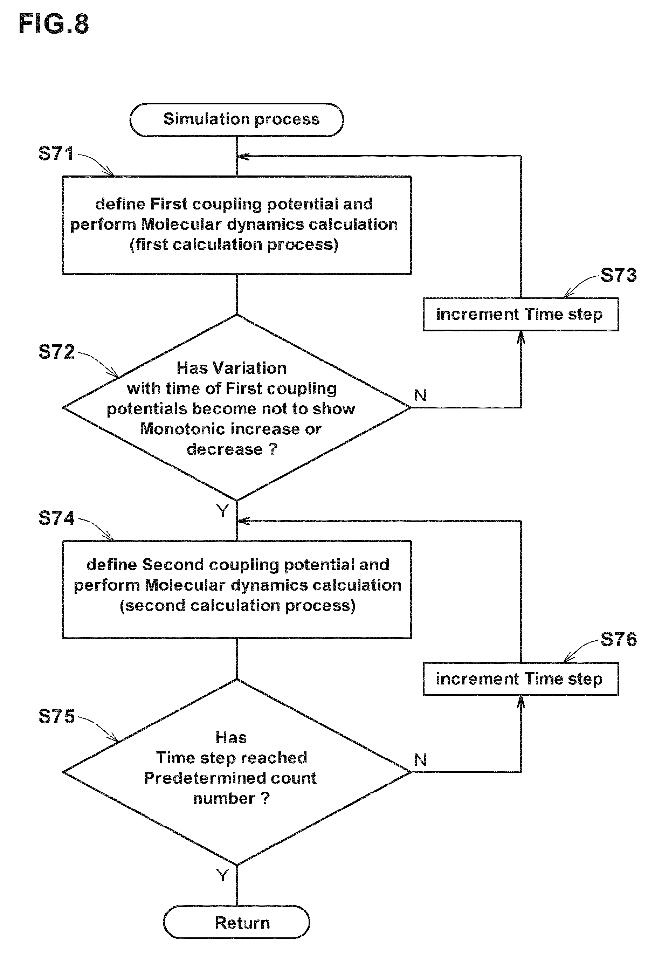

Fig. 8 shows a flowchart of a more specific example of the simulation process S7.

As shown, the simulation process S7 includes a first calculation process S71 and a second calculation process S74 performed thereafter.

In the first calculation process S71, the molecular dynamics calculation is performed under such condition that the above-mentioned first coupling potentials P1 are defined between the particles 4 and 4 and between the particles 4 and particles 8.

In the second calculation process S74, on the other hand, the molecular dynamics calculation is performed under such condition that the above-mentioned second coupling potentials P2 are defined between the particles 4 and 4 and between the particles 4 and particles 8 in order to more accurately simulate the molecular motion of the polymer material.

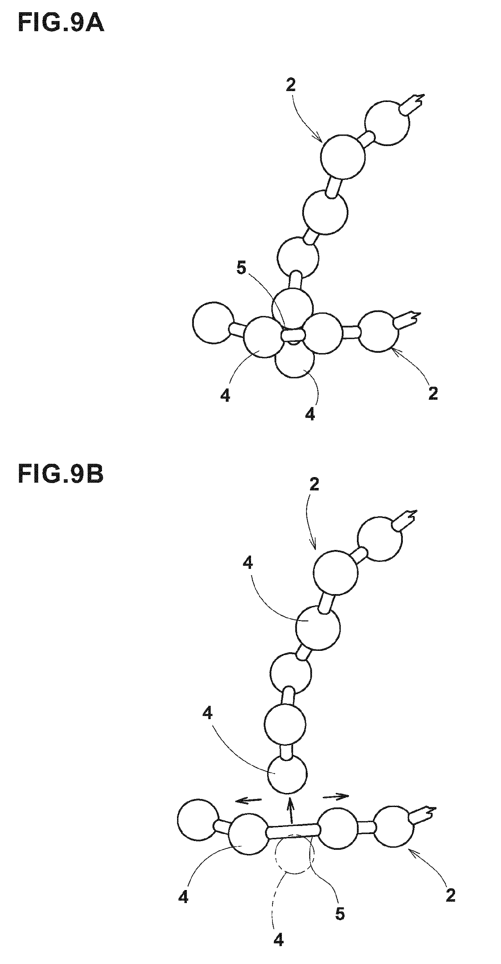

During the initial phase of the relaxation calculation, if the polymer models 2 are arranged in a concentrated manner for example closely to the filler models 12 as shown in Fig. 7, the polymer models 2 (coarse-grained chains) are liable to entangle with each other at the positions between the polymer particles 4 as shown in Fig. 9A.

In the first calculation process S71, since the molecular dynamics calculation is performed under such condition that the interparticle distance $r_{ij}$ is allowed to have values more than the allowable maximum distance $r_0$,

at the positions between the polymer particles 4 at which the polymer models 2 entangle with each other as shown in Fig. 9A, the distances between these polymer particles 4 are readily increased as shown in Fig. 9B, and thereby the polymer models 2 are easily disentangled.

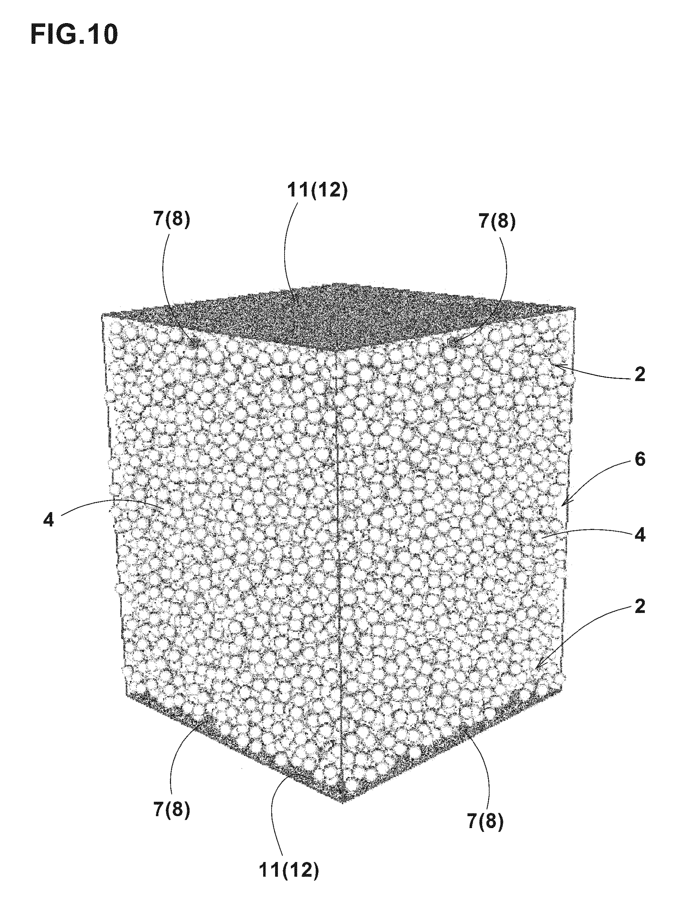

Therefore, in the first calculation process S71, it is possible to avoid calculation failures caused by the acting force excessively-increased by the entanglement between the polymer models 2. As a result, as shown in Fig. 10, the polymer models 2 can be well relaxed without fault.

In the first calculation process S71, it is preferable that a time step for the molecular dynamics calculation is set to be less than the time step suggested by the above-mentioned non-patent document.

In this embodiment, $0.0006\tau$ is set to the time step although $0.006\tau$ is disclosed in the non-patent document. The "$\tau$" is the standard time unit for a Lennard-Jones fluid. Thereby, the amount of traveling of each particle 4, 8 during each time step can be decreased, and as a result, an abrupt increase in the acting force between the polymer models 2 and 2 caused by the entanglement is controlled. This also helps to prevent the occurrence of the calculation failure. In order to effectively derive such effect, the time step is preferably set in a range of from 1/20 to 1/5 of $0.006\tau$. If less than 1/20, there is a possibility that the computational cost increase.

In the next process S72, as shown in Fig. 8, it is judged whether or not the variation with time of the average of the values of all of the first coupling potentials P1 shows a tendency to monotonic increase or decrease.

When the state of the particles 4 and 8 becomes a relaxation state (steady state), the variation with time of the average of the values of all of the first coupling potentials P1 converges in a small range. Therefore, if the variation with time does not show a tendency to monotonic increase or decrease, the state can be judged as having become the relaxation state (steady state).

Therefore, if judged as still showing a tendency to monotonic increase or decrease, then the time step is incremented, and the first calculation process S71 is repeated.

If judged as not showing a tendency to monotonic increase or decrease, the entire process goes to the second calculation process S74.

In the first calculation process S71 in this embodiment, the molecular dynamics calculation is iterated a plural number of times, for example 10 times, while computing the average of the values of all of the first coupling potentials P1 at each time.

If the iteration count has reached to the above-mentioned plural number (10 in this example), the overall mean value of the plural number of the averages is computed.

In this embodiment, therefore, based on such overall mean values obtained until then, namely, by comparing the overall mean values, whether or not the first coupling potentials P1 show a monotonic increase or decrease is determined.

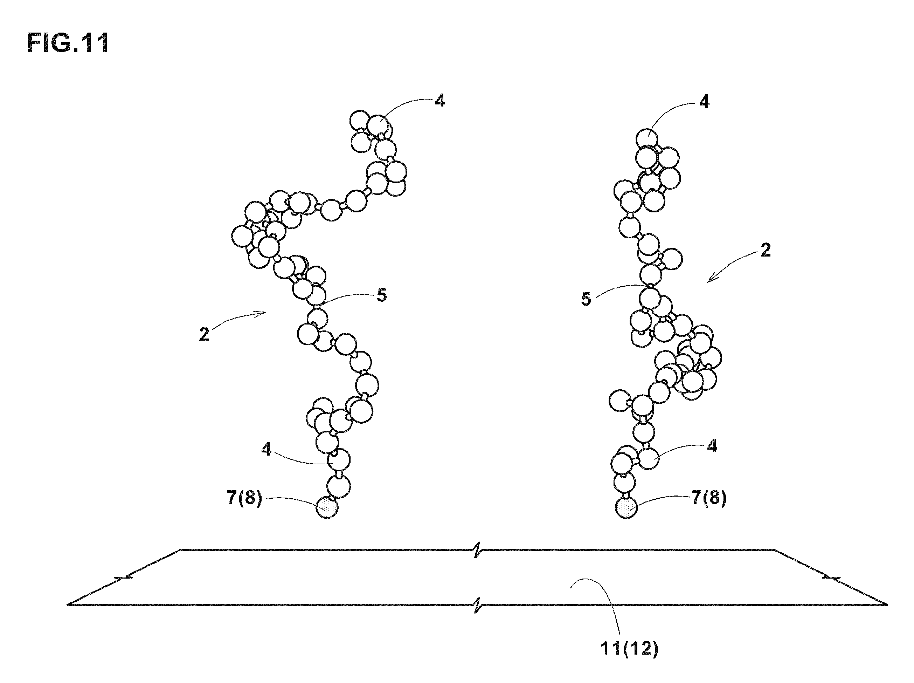

At the time of performing the second calculation process S74, the polymer models 2 have been well relaxed and disentangle through the first calculation process S71.
Under such condition, the molecular dynamics calculation of the second calculation process S74 is performed.
Therefore, the relaxation state of the polymer models 2 can be accurately simulated such that the relaxation state is accurately influenced by the interactive potential T1 between the filler model 12 and polymer particle 4 (nonmodified) and the interactive potential T2 between the filler model 12 and particle 8 (modified).
Thus, the affinity of the modifying agent particles 8 to the filler model 12 (Fig. 11) can be evaluated with accuracy.
In the next process S75, it is judged if the iteration count of the molecular dynamics calculation in the second calculation process S74 has reached to a predetermined number. If not reached yet, the time step is incremented (process S76) and the second calculation process S74 is repeated. If reached, the entire process goes to the next evaluation process S8.
In the evaluation process S8, from the results of the simulation process S7, the relaxation state of the polymer model 2 is assessed. In this example, the affinity of the particle 8 to the filler model 12 is assessed.
In the evaluation process S8, if the relaxation state is judged as being good, the entire process is ended.
If the relaxation state is judged as being not good, the entire process goes to the next process S9.
In the process S9, the conditions previously set to the polymer model 2 and/or filler model 12 for example, are changed, and then, the processes S1 to S7 are again performed.
Thus, it is possible to find out suitable conditions capable of surely relaxing the polymer models 2. These processes S8 and S9 are implemented by humans differently from the above processes S1-S7,
[0079] Although the invention has been described in accordance with the flowchart shown in Fig. 2 with a certain degree of particularity, the flowchart shown in Fig. 2 is just for purposes of illustration or for convenience sake and not to be construed to limit the scope of the invention.
It is understood by those skilled in the art that the most important point is to define the polymer models 2, filler models 12, potentials R, P and T and various conditions before starting the simulation process S7, therefore, the order from S1 to S6 is not essential.
It is to be understood that some of these processes S1-S6 may be performed simultaneously by the computer, and some of these processes S1-S6 may be performed in reverse order.

## Claims

1. A computer implemented method for simulating a polymer material, comprising
   a process in which polymer models of the polymer material are defined,
   a process in which filler models of the filler are defined, and
   a simulation process in which a molecular dynamics calculation is performed on the polymer models and the filler models arranged in a predetermined virtual space,
   wherein
   the polymer model comprises a plurality of polymer particles and bonds between the polymer particles,
   the bond is defined by a coupling potential P,
   the coupling potential P includes a first coupling potential P1 and a second coupling potential P2, the second coupling potential P2 not allowing any interparticle distances more than a predetermined allowable maximum distance, and
   the first coupling potential P1 having no allowable maximum distance for the interparticle distance, and
   the simulation process comprises
   a first calculation process in which the molecular dynamics calculation is performed under such condition that the first coupling potentials P1 are defined between the polymer particles, and
   a subsequent second calculation process in which the molecular dynamics calculation is performed under such condition that the second coupling potentials P2 are defined between the polymer particles.

2. The method to claim 1, wherein
   the first coupling potential P1 is defined by the following equation (1),
   the second coupling potential P2 is defined by the sum of a repulsive potential R defined by the following equation (2) and an attractive potential G defined by the following equation (3):

$$P1 = 0.5k(r_{ij} - r')^2 \qquad --(1)$$

$$P2 = R + G$$

$$R = \begin{cases} 4\varepsilon\left[\left(\dfrac{\sigma}{r_{ij}}\right)^{12} - \left(\dfrac{\sigma}{r_{ij}}\right)^{6} + \dfrac{1}{4}\right] & if\ r_{ij} < 2^{\frac{1}{6}}\sigma \\ 0 & if\ r_{ij} \geq 2^{\frac{1}{6}}\sigma \end{cases} \quad ---(2)$$

$$G = \begin{cases} -0.5kr_0^{\,2}\ln\left[1 - \left(\dfrac{r_{ij}}{r_0}\right)^{2}\right] & if\ r_{ij} < r_0 \\ \infty & if\ r_{ij} \geq r_0 \end{cases} \quad ---(3)$$

wherein
k: the spring constant between the particles concerned,
$r_{ij}$: the interparticle distance between the centers of the particles concerned,
r': the equilibrium distance between the centers of the particles concerned,
$r_0$: a predetermined allowable maximum distance between the centers of the particles concerned.
$\varepsilon$ : a coefficient for the intensity of the repulsive potential R between the particles concerned,
$\sigma$ : a coefficient for adjusting the threshold of the distance $r_{ij}$.

3. The method according to claim 1, wherein
the virtual space has a pair of parallelly-opposed wall surfaces, and
the filler models are respectively defined by the parallelly-opposed wall surfaces.

4. The method according to claim 2, wherein
the virtual space has a pair of parallelly-opposed wall surfaces, and
the filler models are respectively defined by the parallelly-opposed wall surfaces.

# FIG.1

## FIG.2

```
                    ┌─────────────┐
                    │    Start    │
                    └─────────────┘
                           │
S1   ┌─────────────────────────────────────────┐
     │          define Polymer model            │
     └─────────────────────────────────────────┘
                           │
S2   ┌─────────────────────────────────────────┐
     │        define Coupling potential          │
     └─────────────────────────────────────────┘
                           │
S3   ┌─────────────────────────────────────────┐
     │        define Repulsive potential         │
     └─────────────────────────────────────────┘
                           │
S4   ┌─────────────────────────────────────────┐
     │           define Filler model             │
     └─────────────────────────────────────────┘
                           │
S5   ┌─────────────────────────────────────────┐
     │        define Interactive potentials      │
     └─────────────────────────────────────────┘
                           │
S6   ┌─────────────────────────────────────────┐
     │          arrange Polymer models           │
     │            in Virtual space               │
     └─────────────────────────────────────────┘
                           │
S7   ┌─────────────────────────────────────────┐
     │       perform Molecular dynamics          │
     │   calculation (Simulation process)        │
     └─────────────────────────────────────────┘
```

S9 — change Conditions set to Polymer and Filler models

S8 — Is Relaxation state good ?

N

Y

End

# FIG.3

# FIG.4

# FIG.5

FIG.6

# FIG.7

# FIG.8

```
              ┌─────────────────────────┐
              │    Simulation process    │
              └─────────────────────────┘
                           │
S71                        │◄──────────────────────────┐
  ┌──────────────────────────────────────┐             │
  │   define First coupling potential and │             │
  │ perform Molecular dynamics calculation│             │
  │      (first calculation process)      │             │
  └──────────────────────────────────────┘             │
                           │                     S73    │
                           │           ┌────────────────┴──┐
                           │           │ increment Time step│
                           ▼           └───────────────────┘
S72              ╱─────────────────╲                  ▲
            ╱  Has Variation          ╲               │
         ╱  with time of First coupling  ╲      N      │
        ╲  potentials become not to show  ╱ ──────────┘
           ╲   Monotonic increase or    ╱
              ╲    decrease ?         ╱
                  ╲──────────────╱
                        │ Y
S74                     │◄──────────────────────────┐
  ┌──────────────────────────────────────┐          │
  │  define Second coupling potential and │          │
  │ perform Molecular dynamics calculation│          │
  │      (second calculation process)     │          │
  └──────────────────────────────────────┘          │
                           │                   S76    │
                           │         ┌────────────────┴──┐
                           │         │ increment Time step│
                           ▼         └───────────────────┘
S75              ╱─────────────────╲                ▲
            ╱       Has                ╲             │
         ╱    Time step reached          ╲    N      │
        ╲   Predetermined count          ╱ ─────────┘
           ╲      number ?             ╱
              ╲──────────────────────╱
                        │ Y
              ┌─────────────────────────┐
              │         Return          │
              └─────────────────────────┘
```

FIG.9A

FIG.9B

**FIG.10**

FIG.11

**FIG.12A**

**FIG.12B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Dynamics of entangled linear polymer melts; A molecular-dynamics simulation. *Journal of Chemical Physics,* 1990, vol. 92, 5057